Europäisches Patentamt

(19) European Patent Office

Office européen des brevets

(11) Publication number: 0 183 245
A2

(12) **EUROPEAN PATENT APPLICATION**

(21) Application number: 85115060.7

(22) Date of filing: 27.11.85

(51) Int. Cl.⁴: **C 07 K 5/08**
C 07 K 5/10, A 23 K 1/16

(30) Priority: 30.11.84 GB 8430255

(43) Date of publication of application:
04.06.86 Bulletin 86/23

(84) Designated Contracting States:
AT BE CH DE FR GB IT LI LU NL SE

(71) Applicant: FARMITALIA CARLO ERBA S.p.A.
Via Carlo Imbonati n.24
I-20159 Milan(IT)

(72) Inventor: Hecht, Barbare
Via Barzini, 14
I-20125 Milan(IT)

(72) Inventor: Perseo, Giuseppe
Via Monte Bianco, 66
I 20033 Desio (Milan)(IT)

(72) Inventor: de Castiglione, Roberto
Via Domeinchino, 38
I-20149 Milan(IT)

(74) Representative: Lehn, Werner, Dipl.-Ing. et al,
Hoffmann, Eitle & Partner Patentanwälte Arabellastrasse
4 (Sternhaus)
D-8000 München 81(DE)

(54) Animal growth promotant.

(57) An animal growth promotant which comprises as an effective ingredient a peptide of the following formula I:

$$X-A-B-Trp-C-Y$$

wherein
X is selected between hydrogen and t-butoxycarbonyl;
A is selected from the group consisting of Leu, Ile, Phe, Ahx, Met, MetO and Phe(Cl), either of L- or D-configuration;
B is selected from the group consisting of Pro, Ala, Gly, Val, Leu and Pip, either of L- or D-configuration;
C is selected from the group consisting of Val, Leu, Met, MetO, Ile, Phe, Phe(Cl) and Ahx, either of L- or D-configuration; or A, B and C independently represent a chemical bond provided that at least one of them is an amino acid residue;
Y is selected from the group consisting of OH, OMe, OEt, OBzl, $NH_2$, NH-Et, $NEt_2$
and the veterinarily acceptable salts thereof.
A feed or veterinary composition containing it, and a method for promoting the growth of animals are also provided.

New compounds according to formula I, wherein A is different from L-Leu or L-Phe or C is a chemical bond, as well as a method for preparing the same is disclosed.

Croydon Printing Company Ltd.

## TITLE OF THE INVENTION

"ANIMAL GROWTH PROMOTANT"

## DESCRIPTION

This invention relates to a new animal growth promotant. More particularly, it relates to a new composition which comprises a peptide or its non toxic salt as an effective ingredient, and to methods for promoting the growth of animals and improving the efficency of feed utilization by animals, which comprises the administration of the composition comprising the peptide or its non toxic salt to animals.

The peptide to be used in this invention is represented by the following formula (I) :

$$X-A-B-Trp-C-Y$$

wherein

X  is selected between hydrogen and t-butoxycarbonyl;

A  is selected from the group consisting of Leu, Ile, Phe, Ahx, Met, MetO and Phe(Cl), either of L- or D-configuration;

B  is selected from the group consisting of Pro, Ala, Gly, Val, Leu and Pip, either of L- or D-configuration;

C  is selected from the group consisting of Val, Leu, Met, MetO, Ile, Phe, Phe(Cl) and Ahx, either of L- or D-configuration; or A, B and C independently represent a chemical bond provided that at least one of them is an amino acid residue;

Y  is selected from the group consisting of OH, OMe, OEt, OBzl, $NH_2$, NH-Et, $NEt_2$

and veterinarily acceptable salts thereof.

Moreover, the present invention relates to the new peptides of the general formula (I) wherein either C is a chemical bond or A is different from L-Leu or L-Phe and to the following peptides :

H-Leu-Gly-Trp-Met-OH

H-Leu-Pro-Trp-Met-OH

H-Leu-Pro-Trp-Met-OMe

H-Leu-Pro-Trp-Met-NH$_2$

H-Leu-Ala-Trp-Met-OH

H-Leu-Ala-Trp-Met-OMe

H-Leu-Ala-Trp-Met-NH$_2$

H-Leu-D-Ala-Trp-Met-OH

H-Leu-D-Ala-Trp-Met-OMe

H-Leu-D-Ala-Trp-Met-NH$_2$

H-Leu-Ala-Trp-Ahx-OH

H-Leu-Ala-Trp-Ahx-OMe

H-Leu-Ala-Trp-Ahx-NH$_2$

H-Leu-Gly-Trp-Phe-OH

H-Leu-Gly-Trp-Phe-OMe

H-Leu-Gly-Trp-Phe-NH$_2$

The other compounds of structure (I) are known and described just as intermediates in GB 2130590 A and Europ. Pat.Appln. No.0134986 A.

Salts of peptides according to the invention with veterinarily acceptable acids or bases are within the scope of the invention. Such acid addition salts can be derived from a variety of inorganic and organic acids such as sulphuric, phosphoric, hydrochloric, hydrobromic, hydroiodic, nitric, sulfamic, citric, lactic, piruvic, oxalic, maleic, succinic, tartaric, cinnamic, acetic, trifluoroacetic, benzoic, salicilic, gluconic, ascorbic and related acids.

Such base addition salts can be derived from a variety of inorganic and organic bases such as sodium hydroxide, potassium hydroxide, diethylamine, triethylamine, dicyclohexylamine.

The synthesis of the new peptides is accomplished by classical solution methods and is in the scope of the invention.

The synthesis consists essentially in appropriate successive condensations of protected amino acids or peptides. The condensation is carried out so that the resulting peptides have the desired sequence of two to four amino acid residues. The amino acids and peptides, which are condensed according to methods known in themselves in polypeptide chemistry, have their amino and carboxyl groups, which are not involved in the formation of the peptide linkage, blocked by a suitable protecting groups.

The protecting groups are capable of being removed by acidolysis, saponification and hydrogenolysis. For the protection of the amino groups the following protective groups may, for example, be employed: benzyloxycarbonyl, t-butoxycarbonyl, trityl, formyl, trifluoroacetyl, o-nitrophenylsulphenyl, 4-methoxybenzyloxycarbonyl, 9-fluorenylmethoxycarbonyl, 3,5-dimethoxy-$\alpha,\alpha'$-dimethylbenzyloxycarbonyl or methylsulphonylethoxycarbonyl.

For the protection of the carboxyl groups the following protective groups may, for example, be employed: methyl, ethyl, t-butyl, benzyl, p-nitrobenzyl or fluorenylmethyl.

The condensation between an amino group of one molecule and a carboxyl group of another molecule to form the peptidic linkage may be carried out through an activated acyl-derivative such as a mixed anhydride, an azide or activated ester, or by direct condensation between a free amino group and a free carboxyl group, in the presence of a condensing agent such as dicyclohexylcarbodiimide, alone or together with a racemization preventing agent, such as N-hydroxysuccinimide or 1-hydroxybenzotriazole.

The condensation may be carried out in a solvent such as dimethylformamide, pyridine, acetonitrile, tetrahydrofuran or N-methyl-2-pyrrolidone. The reaction temperature may be from -30°C to ambient temperature. The reaction time is generally from 1 to 120 hours. The scheme of synthesis, the protecting groups and the condensing agents are selected so as to avoid the risk of racemization.

De-protecting reactions are carried out according to methods known per se in polypeptide chemistry. Peptides wherein Y represents OMe, OEt or OBzl are prepared, for example, starting from the C-terminal amino acid esterified by the appropriate alcohol. Peptides wherein Y represents OH can be prepared, for example, by hydrolysis of peptides wherein Y represents OMe or OEt, or by hydrogenolysis of peptides wherein Y represents OBzl.

Peptides wherein Y represents $NH_2$, NHEt or $NEt_2$ can be prepared by ammonolysis of the corresponding esters or starting from a C-terminal amino acid amidated by the appropriate amine.

The final condensation in the preparation of a peptide according to the invention is preferably between a compound of formula II:

$$X-A-B-OH$$

in the presence of a condensing agent such as dicyclohexylcarbodiimide, alone or together with a racemization preventing agent, or a mixed anhydride, activated ester or azide derivative of the compound of formula II as defined above, and a compound of formula III:

$$H-Trp-C-Y$$

wherein A, B, C, X and Y are as herein defined save that X does not represent a hydrogen atom and Y does not represent a OH group. The compounds according to the invention show an interesting growth promoting activity in animals determined both by the in vivo - in vitro test system on protein synthesis of liver tissue (as described by K. Kämmerer and A. Dey-Hazra (1980) Vet. Med. Nachr. Nr. 2, 99-112), and by the dose-dependent increase in weight gain and feed efficiency after subcutaneous or oral administration. The peptides of general formula I, described just as intermediate in the previously cited documents, unlike the corresponding end-compounds have lost the activity on the endocrinological and nervous system but, as stated above, have completely retained the capability to induce growth in animals.

In vivo - in vitro test - Protein synthesis

The test was carried out for periods of 7 days using groups of 6 male rats (Wistar, Hagemann, Extertal) divided in subgroups of three animals managed in Makrolon cages, wood shaving as litter.

Water and feed (Altromin 1321 Standard diet containing 19% crude protein): ad libitum.

The peptides of the present invention were administered in solution s.c. daily at doses ranging from 0.1 to 100 ng/kg, using as diluent normal saline (starting from a stock solution with 100 ng/ml).

## Preparation of Tissue Samples

Homogenise 3 g liver in 9 ml TKM buffer-saccharose solution, cooled on ice, in a Potter homogeniser at 600 r.p.m. for 2 min.; centrifuge at 4°C in an ultracentrifuge with 10,000 g for 20 min., decant the supernatant- microsomal cell juice.

## Working procedure

After calculation of protein content in the microsomal cell juice by means of the biuret method, the protein concentration was adjusted with TKM buffer to 1 mg/ml. To that, a further dilution with bidistilled water to 0.25 mg protein per ml of the microsomal cell juice followed.

Subsequently portions of 0.15 ml reaction medium and 0.05 ml (50 mcg) of pyruvate kinase solution, as well as 0.1 ml of $^{14}C$ amino acid mixture (= 1 μCi), were added. The volume of the incubation mixture was then 1 ml each. After a 35 minute incubation at 37°C in a water bath, protein precipitation was performed by adding 2 ml of trichloracetic acid (10%). The sediment was washed by several additions of trichloroacetic acid and subsequent centrifugation (3600 g/5 min) until the supernatant was free from radioactivity. The residue was dissolved in 1.0 ml Lumasolve and left overnight at 37°C, until it was clear.

Measuring of the preparation was done in a PRIAS liquid scintillation counter PL (1.0 ml + 5 ml scintillation liquid).

A detailed method is described in the above cited Kämmerer's article.

The measured protein synthesis rates of the compounds listed in Table 1 are significantly different from the control group constituted by rats treated with daily s.c. administration of physiological sodium chloride solution.

TABLE 1 - Results of the measurements of the protein synthesis rates of
liver tissues of male rats after treatment for a period of
7 days (daily s.c. injections).

| Treatment with | Doses[1] (ng/kg) | CPM | Δ% treated versi control rat gri |
|---|---|---|---|
| Boc-Trp-Leu-NH$_2$ Int. J. Peptide Protein Res. 24, 155 (1984) | 6 30 60.1 | 5035 6874 6204 | - 3.5 + 31.7 + 18.9 |
| HCl . H-Trp-Leu-NH$_2$ Int. J. Peptide Protein Res. 24, 155 (1984) | 4.6 22.8 45.5 | 6857 6425 5303 | + 31.4 + 23.1 + 1.6 |
| HCl . H-Trp-Val-OMe Example 3, US 4491541 | 6.2 -- 61.9 | 5081 -- 5145 | + 42.3 -- + 44.1 |
| HCl . H-Pro-Trp-Met-NH$_2$ Example 2, US 4491541 | 6.2 31.1 62.3 | 9518 8608 7977 | + 18.9 + 7.5 - 0.3 |
| HCl . H-Leu-Gly-Trp-Met-NH$_2$ Int. J. Peptide Protein Res. 25, 316 (1985) | 6.3 31.6 63.2 | 4392 4711 4628 | + 23.0 + 32.0 + 29.6 |
| HCl . H-Leu-Gly-Trp-OMe Compound IV | 10 50 100 | 365 518 563 | -- + 11.9 + 21.6 |
| HCl . H-Ahx-Gly-Trp-OMe Compound VIII | 10 50 100 | 606 584 547 | + 30.9 + 26.1 + 18.1 |
| HCl . H-Trp-Phe-OMe Compound XII | 10 50 100 | 509 529 536 | + 1.6 + 5.6 + 7.0 |
| HCl . H-Ahx-Gly-Trp-Phe-OMe Compound XIV | 10 50 100 | 523 578 546 | + 4.4 + 15.4 + 9.0 |

1) Doses are related to peptides free bases.

In the evaluation of growth promoting activity _in vivo_, rats with a starting weight of about 79 g were treated orally through feed (Altromin 1320) at doses ranging from 4 to 250 ng/kg body weight. Weight gain and feed intake were recorded weekly.

The results are shown in the tables below (Doses of salified peptides are referred to the free bases).

| PEPTIDE: Boc-Trp-Leu-NH$_2$ | | | |
|---|---|---|---|
| DOSES (ng/kg body weight) | 6.0 mean ± sd | 30.0 mean ± sd | 60.1 mean ± sd |
| Initial weight | 79.0 ± 8.7 | 79.0 ± 4.7 | 78.7 ± 7.0 |
| Final weight | 238.5 ± 10.5 | 236.5 ± 11.6 | 237.3 ± 11.6 |
| Weight gain (absolute) | 159.5 | 157.5 | 158.6 |
| Weight gain (% respect to control) | +4.86 | +3.55 | +4.27 |
| Feed consumption (g) | 559.7 | 530.2 | 574.6 |
| Feed conversion (g) (absolute) | 3.50 | 3.37 | 3.62 |
| Feed conversion (% respect to control) | + 4.37 | + 7.92 | + 1.09 |

PEPTIDE: HC1 . H-Trp-Leu-NH$_2$

| DOSES<br>(ng/kg body weight) | 4.6<br>mean ± sd | 22.8<br>mean ± sd | 45.5<br>mean ± sd |
|---|---|---|---|
| Initial weight | 78.0 ± 4.6 | 78.8 ± 3.1 | 79.2 ± 6.1 |
| Final weight | 238.0 ± 9.9 | 238.5 ± 8.8 | 239.9 ± 13.5 |
| Weight gain (absolute) | 159.4 | 159.7 | 160.7 |
| Weight gain (% respect to control) | + 4.80 | + 5.00 | + 5.65 |
| Feed consumption (g) | 568.4 | 536.2 | 558.5 |
| Feed conversion (g) (absolute) | 3.56 | 3.56 | 3.47 |
| Feed conversion (% respect to control) | + 2.73 | + 2.73 | + 5.19 |

PEPTIDE: HC1 . H-Trp-Val-OMe

| DOSES<br>(ng/kg body weight) | 61.9<br>mean ± sd | 123.8<br>mean ± sd | 247.6<br>mean ± sd |
|---|---|---|---|
| Initial weight | 79.8 ± 5.6 | 78.5 ± 6.1 | 79.8 ± 4.7 |
| Final weight | 246.8 ± 15.4 | 236.4 ± 17.7 | 236.2 ± 12.8 |
| Weight gain (absolute) | 167.0 | 157.9 | 156.3 |
| Weight gain (% respect to control) | + 9.80 | + 3.81 | + 2.76 |
| Feed consumption (g) | 549.9 | 569.2 | 554.2 |
| Feed conversion (g) absolute) | 3.29 | 3.60 | 3.54 |
| Feed conversion (% respect to control) | +10.11 | + 1.64 | + 3.28 |

PEPTIDE: HCl . H-Leu-Gly-Trp-Met-NH$_2$

| DOSES (ng/kg body weight) | 31.6 mean ± sd | 63.3 mean ± sd | 126.6 mean ± sd |
|---|---|---|---|
| Initial weight | 79.9 ± 4.4 | 78.0 ± 3.8 | 79.4 ± 5.5 |
| Final weight | 243.8 ± 21.1 | 255.7 ± 6.5 | 227.5 ± 11.5 |
| Weight gain (absolute) | 163.9 | 177.7 | 148.1 |
| Weight gain (% respect to control) | + 7.76 | +16.83 | - 2.63 |
| Feed consumption (g) | 564.8 | 563.4 | 564.2 |
| Feed conversion (g) (absolute) | 3.45 | 3.17 | 3.69 |
| Feed conversion (% respect to control) | + 5.74 | +13.39 | - 0.28 |

| CONTROL | mean + sd |
|---|---|
| Initial weight | 78.0 + 5.5 |
| Final weight | 230.0 + 14.3 |
| Weight gain (absolute) | 152.1 |
| Feed consumption (g) | 556.9 |
| Feed conversion (g) (absolute) | 3.66 |

0183245

It is another embodiment of this invention to provide a method for promoting the growth of animals and improving their feed efficiency by administering to them a compound of this invention. This method will be particularly suitable for animals raised for food such as fowl, ruminants, swine and rabbits.

Although all members of the fowl family - i.e. chickens, turkeys, geese, ducks, guinea fowl, pheasant and quail - show increased rate of growth and improved feed efficiency, the method is particularly valuable for broiler chickens and turkeys.

Of the ruminants, e.g. cattle, sheep and goats, the method is particularly of value for cattle, e.g. steers.

A method of administration of a compound of this invention is to incorporate it in the feed rations intended for the animal at a concentration from about 0.1 to 100 mcg/kg of feed, preferably from about 0.1 to 10 mcg/kg. The animals are permitted to feed at liberty throughout the growth period. There are many specialized feed rations for different species of animals. The compounds of this invention can be used with any of the known rations. The term "feed rations" is intended to mean the food provided for the animals, and it is not intended that the invention be limited thereby. Preferably the compound is thoroughly mixed with the feed ration so that it is uniformly dispersed throughout. Any of the known feed rations can be used in the practice of this invention and it is not intended that the invention be limited by the formulation of the ration.

Feed rations are formulated to provide the animals for which they are intended with the essential nutrients, minerals, vitamins, bulk, etc. Formulations of these rations are well within the skill of nutritionists.

Another method for administering the compounds of the present invention is by means of a subcutaneous implant, e.g. in a pellet form of the peptides of the present invention to be subcutaneously injected to the animals with a release of substance per day in a range from 0.1 to 300 ng/kg, preferably from about 1 to 100 ng/kg.

Thus, it is not intended that the invention be limited to any particular mode of administration.

Accordingly, the invention further provides a veterinary composition comprising a compound of the invention or a veterinarily acceptable salt thereof in admixture with a veterinarily acceptable diluent or carrier; in addition, these preparations can have directed or delayed liberation of the active ingredient.

Veterinarily acceptable carrier refers to an edible material to which the peptides of the invention are added to facilitate uniform incorporation of such peptides into feeds.

The active peptide is adsorbed, impregnated or coated into or onto the edible material in such a way as to disperse and physically carry  the active peptide.

The drinking water comprising the compounds of the present invention can be prepared by simply dissolving the compounds of general formula I  or their salts in water in a proper amount.

0183245

Representative compounds of the peptide (I) to be used in the present invention are as follows :

Boc-Trp-Leu-OH

Boc-Trp-Leu-OMe

Boc-Trp-Leu-NH$_2$

H-Trp-Leu-OH

H-Trp-Leu-OMe

H-Trp-Leu-NH$_2$

Boc-Trp-Val-OH

Boc-Trp-Val-OMe

Boc-Trp-Val-NH$_2$

H-Trp-Val-OH

H-Trp-Val-OMe

H-Trp-Val-NH$_2$

Boc-Pro-Trp-Met-OH

Boc-Pro-Trp-Met-OMe

Boc-Pro-Trp-Met-NH$_2$

H-Pro-Trp-Met-OH

H-Pro-Trp-Met-OMe

H-Pro-Trp-Met-NH$_2$

Boc-Leu-Gly-Trp-Met-OH

Boc-Leu-Gly-Trp-Met-OMe

Boc-Leu-Gly-Trp-Met-NH$_2$

H-Leu-Gly-Trp-Met-OH

H-Leu-Gly-Trp-Met-OMe

H-Leu-Gly-Trp-Met-NH$_2$

The following representative peptides are also new :

H-D-Leu-Gly-Trp-Met-OH

H-D-Leu-Gly-Trp-Met-OMe

H-D-Leu-Gly-Trp-Met-NH$_2$

H-Leu-Gly-Trp-Met-OH

H-Leu-Pro-Trp-Met-OH

H-Leu-Pro-Trp-Met-OMe

H-Leu-Pro-Trp-Met-NH$_2$

H-Leu-Ala-Trp-Met-OH

H-Leu-Ala-Trp-Met-OMe

H-Leu-Ala-Trp-Met-NH$_2$

H-Leu-D-Ala-Trp-Met-OH

H-Leu-D-Ala-Trp-Met-OMe

H-Leu-D-Ala-Trp-Met-NH$_2$

H-Leu-Ala-Trp-Ahx-OH

H-Leu-Ala-Trp-Ahx-OMe

H-Leu-Ala-Trp-Ahx-NH$_2$

H-D-Leu-Gly-Trp-Ahx-OH

H-D-Leu-Gly-Trp-Ahx-OMe

H-D-Leu-Gly-Trp-Ahx-NH$_2$

H-Phe(Cl)-Gly-Trp-Met-OH

H-Phe(Cl)-Gly-Trp-Met-OMe

H-Phe(Cl)-Gly-Trp-Met-NH$_2$

H-D-Phe-Gly-Trp-Met-OH

H-D-Phe-Gly-Trp-Met-OMe

H-D-Phe-Gly-Trp-Met-NH$_2$

H-Ahx-Gly-Trp-Met-OH

H-Ahx-Gly-Trp-Met-OMe

H-Ahx-Gly-Trp-Met-NH$_2$

H-Leu-Gly-Trp-Phe-OH

H-Leu-Gly-Trp-Phe-OMe

H-Leu-Gly-Trp-Phe-NH$_2$

H-Ahx-Gly-Trp-Phe-OH

H-Ahx-Gly-Trp-Phe-OMe

H-Ahx-Gly-Trp-Phe-NH$_2$

H-Phe(Cl)-Gly-Trp-Phe-OH

H-Phe(Cl)-Gly-Trp-Phe-OMe

H-Phe(Cl)-Gly-Trp-Phe-NH$_2$

H-Ahx-Gly-Trp-Val-OH

H-Ahx-Gly-Trp-Val-OMe

H-Ahx-Gly-Trp-Val-NH$_2$

H-Ahx-Ala-Trp-Val-OH

H-Ahx-Ala-Trp-Val-OMe

H-Ahx-Ala-Trp-Val-NH$_2$

H-Ala-Trp-Met-OH

H-Ala-Trp-Met-OMe

H-Ala-Trp-Met-NH$_2$

H-Ala-Trp-Phe-OH

H-Ala-Trp-Phe-OMe

H-Ala-Trp-Phe-NH$_2$

H-Ala-Trp-Leu-OH

H-Ala-Trp-Leu-OMe

0183245

H-Ala-Trp-Leu-NH$_2$

H-Ala-Trp-Val-OH

H-Ala-Trp-Val-OMe

H-Ala-Trp-Val-NH$_2$

H-D-Ala-Trp-Met-OH

H-D-Ala-Trp-Met-OMe

H-D-Ala-Trp-Met-NH$_2$

H-D-Ala-Trp-Met-NH$_2$

H-Leu-Gly-Trp-OH

H-Leu-Gly-Trp-OMe

H-Leu-Gly-Trp-NH$_2$

H-Leu-D-Ala-Trp-OH

H-Leu-D-Ala-Trp-OMe

H-Leu-D-Ala-Trp-NH$_2$

H-D-Leu-Gly-Trp-OH

H-D-Leu-Gly-Trp-OMe

H-D-Leu-Gly-Trp-NH$_2$

H-Ahx-Gly-Trp-OH

H-Ahx-Gly-Trp-OMe

H-Ahx-Gly-Trp-NH$_2$

H-Ahx-D-Ala-Trp-OH

H-Ahx-D-Ala-Trp-OMe

H-Ahx-D-Ala-Trp-NH$_2$

H-D-Ahx-Gly-Trp-OH

H-D-Ahx-Gly-Trp-OMe

H-D-Ahx-Gly-Trp-NH$_2$

The following Examples are intended to illustrate the invention without limiting it. The Rf values were determined on pre-coated plates of silica gel 60 $F_{254}$ (Merck) layer thickness 0.25 mm, length 20 cm, using the following development systems:

System A: benzene/benzine (60-80)/ethyl acetate= 70/10/40 by volume

System B: benzene/ethyl acetate/acetic acid/water= 100/100/20/10 by volume (upper phase)

System C: benzene/ethyl acetate/acetic acid/water= 100/100/40/15 by volume (upper phase)

System D: n-butanol/acetic acid/water= 4/1/1 by volume

System E: methylene chloride/methyl alcohol= 98/2 by volume

System F: methylene chloride/methyl alcohol= 05/5 by volume

"E. Merck" is a trade mark.

TLC analysis were carried out at a temperature ranging from 18 to 25°C: the $R_f$ values can therefore change ± 5%. Melting points are determined in open capillaries with a Tottoli apparatus and are uncorrected. Most of the derivatives soften and decompose before melting. High voltage paper electrophoresis is carried out with a Pherograph-Original-Frankfurt Type 64 apparatus on Schleicher and Schüll paper n. 2317 at pH 1.2 (formic acid:acetic acid:water= 123:100:777) at 1600 V (40 V/cm), and at pH 5.8 (pyridine:acetic acid:water= 450:50:4500) at 1400 V (32.5 V/cm). The products were characterized by their mobilities relative to Glu at pH 1.2 ($E_{1.2}$), and at pH 5.8 ($E_{5.8}$).

In this specification symbols and abbreviations are those commonly used in peptide chemistry (see Eur. J. Biochem. (1984) 138, 9-37). Other symbols and abbreviations used are: AcOEt, ethyl acetate; DMF, dimethylformamide; Et$_2$O, diethyl ether; MeOH, methyl alcohol; NMM, N-methyl-morpholine; PE, petroleum ether; Phe(Cl), p-chloro-L-phenyl alanine; Pip, L-pipecolic acid; iPr$_2$O, diisopropyl ether; iPrOH, isopropyl alcohol; THF, tetrahydrofuran.

## Example 1

Preparation of HCl . H-Leu-Gly-Trp-OMe (IV)

Step 1.  Boc-Leu-Gly-OBzl (I)

To a solution of 4.63g (20 mmol) of Bcc-Leu-OH in 60 ml of anhydrous THF, 2.2 ml (20 mmol) of NMM and 1.98 ml (20 mmol) of ethylchloroformate were successively added at a temperature of -12°C.

After stirring at this temperature for 2 minutes, a cold solution (-30°C) of 6.75 g (20 mmol) of H-Gly-OBzl tosylate and 2.2 ml of NMM (20 mmol) in 60 ml of DMF was added.

The reaction mixture was stirred for 45 minutes at -12°C and for 90 minutes at 0-15°C, then filtered from salts and evaporated in vacuo.

The residue was dissolved in ethyl acetate and washed several times successively with sodium chloride saturated solutions of 1 M citric acid, 1 M sodium bicarbonate and water.  The organic layer was dried over anhydrous sodium sulphate and the solvent removed in vacuo.

6.81 g (90% yield) of compound I were obtained as an oil.

Rf$_E$ 0.59.

Step 2.  Boc-Leu-Gly-OH (II)

6.6 g (17.44 mmol) of Boc-Leu-Gly-OBzl (I)  dissolved in 50 ml of
methanol were hydrogenated at room temperature and atmospheric pres-
sure in the presence of 1.65 g of 10% palladium-on-charcoal.   The
catalyst was removed by filtration and the solution was concentrated
in vacuo.

4.17 g (83% yield) of compound II were obtained from AcOEt/PE;
m.p. 115-121°C; $[\alpha]_D^{26}$ - 29.0° (c= 1 MeOH); $Rf_B$ 0.50; $E_{5.8}$ 0.75.


Step 3.  Boc-Leu-Gly-Trp-OMe (III)

Starting from 3.95 g (13.70 mmol) of Boc-Leu-Gly-OH (II) and 3.49·g
(13.70 mmol) of HCl . H-Trp-OMe  and operating as described in Step 1,
6.02 g (90% yield) of partially purified compound III were obtained
as a foam from $Et_2O$ ($Rf_A$ 0.39; $Rf_B$ 0.69) and used as such in the next
Step.

Step 4.   HCl . H-Leu-Gly-Trp-OMe (IV)

5.75 g (11.77 mmol) of Boc-Leu-Gly-Trp-OMe (III) were dissolved in
60 ml of a saturated solution of hydrogen chloride in glacial acetic
acid to which 6 ml of anisole and 3 ml of 2-mercaptoethanol were added.
After 30 minutes at room temperature the Boc-removal was complete and
the solvent removed in vacuo at 30°C.   The crude product was purified
by column chromatography on silica gel (Merck) 0.040-0.063 mm, eluting
with ethyl acetate:methanol= 9:1.

From $AcOEt/Et_2O$ 3.05 g (61% yield) of compound IV were obtained: m.p.
180-185°C (foam); $[\alpha]_D^{26}$ + 16.9° (c= 1, MeOH); $Rf_D$ 0.67; $E_{1.2}$ 0.80.

Example 2

Preparation of HCl . H-Ahx-Gly-Trp-OMe (VIII)

Step 1.   Boc-Ahx-Gly-OBzl (V)

Starting from 4.63 g (20 mmol) of Boc-Ahx-OH and 6.75 g (20 mmol) of H-Gly-OBzl tosylate and operating as described in Example 1, Step 1, 6.66 g (88% yield) of compound V were obtained from AcOEt/PE: m.p. 84-86°C; $[\alpha]_D^{26}$ - .22.0° (c= 1, MeOH); $Rf_E$ 0.58.

Step 2.   Boc-Ahx-Gly-OH (VI)

Starting from 6.35 g (16.78 mmol) of Boc-Ahx-Gly-OBzl (V) and operating as described in Example 1, Step 2, 4.31 g (89% yield) of compound VI were obtained as an oil: $Rf_B$ 0.54; $E_{5.8}$ 0.74.

Step 3.   Boc-Ahx-Gly-Trp-OMe (VII)

Starting from 4.12 g (14.28 mmol) of Boc-Ahx-Gly-OH (VI) and 3.64 g (14.28 mmol) of HCl . H-Trp-OMe   .                         and operating . ' as described in Example 1, Step 1, 5.93 g (85% yield) of compound VII were obtained as on oil: $Rf_B$ 0.81.

Step 4.   HCl . H-Ahx-Gly-Trp-OMe (VIII)   .

Starting from 5.60 g (11.46 mmol) of Boc-Ahx-Gly-Trp-OMe (VII) and operating as described in Example 1, Step 4, but using methylene chloride containing increasing amount of methyl alcohol (up to 5%) as eluent in the purification step, 3.61 g (65% yield) of compound VIII were obtained from AcOEt/Et$_2$O: m.p. 114-117°C (foam); $[\alpha]_D^{25}$ + 25.3° (c= 1 MeOH) $Rf_D$ 0.64; $E_{1.2}$ 0.79.

Example 3

Preparation of HCl . H-Ahx-Gly-Trp-Met-OMe (X)

Step 1.   Boc-Ahx-Gly-Trp-Met-OMe (IX)

Starting from 1.66 g (5.41 mmol) of Boc-Ahx-Gly-OH (VI, Example 2) and 2.09 g (5.41 mmol) of HCl . H-Trp-Met-OMe (G. Perseo et al. Int. J. Peptide Protein Res. (1985) 25, 316-322) and operating as described in Example 1, Step 1, but using methylene chloride in the purification step, 2.85 g (87% yield) of compound IX were obtained from AcOEt/Et$_2$O: Rf$_B$ 0.74.

Step 2.   HCl . H-Ahx-Gly-Trp-Met-OMe   (X)

Starting from 2.60 g (4.19 mmol) of compound IX and operating as described in Example 1, Step 4, but using ethyl acetate containing increasing amount of methyl alcohol (from 10 to 15%) as eluent in the purification step, 1.40 g (60% yield) of compound X were obtained from iPrOH/Et$_2$O:  m.p. 66-70°C (foam) $[\alpha]_D^{25}$- 5.9° (c= 1, MeOH); Rf$_D$ 0.70; E$_{1.2}$ 0.69.

Example 4

Preparation of HCl . H-Ahx-Gly-Trp-Phe-OMe (XIV)

Step 1.   Boc-Trp-Phe-OMe (XI)

Starting from 6.09 g (20 mmol) of Boc-Trp-OH and 4.31 g (20 mmol) of HCl . H-Phe-OMe and operating as described in Example 1, Step 1, 7.73 g (83% yield) of compound XI were obtained from AcOEt/iPr$_2$0: m.p. 117-120°C; $[\alpha]_D^{25}$ - 20.2° (c= 1 MeOH); Rf$_A$ 0.79; Rf$_F$ 0.56.

Step 2.   HCl . H-Trp-Phe-OMe (XII)

Starting from 7.47 g (16.05 mmol) of compound XI and operating as described in Example 1, Step 2, 5.55 g (86% yield) of compound XII were obtained from AcOEt/Et$_2$0:  Rf$_0$ 0.75, E$_{1.2}$ 0.83.

Step 3.   Boc-Ahx-Gly-Trp-Phe-OMe (XIII)

Starting from 1.83 g (6.35 mmol) of Boc-Ahx-Gly-OH (VI) and 2.55 g (6.35 mmol) HCl . H-Trp-Phe-OMe (XII), and operating as described in Example 3, Step 1, 3.19 g (79% yield) of compound XIII were obtained as an oil: Rf$_C$ 0.76.

Step 4.   HCl . H-Ahx-Gly-Trp-Phe-OMe (XIV)

Starting from 2.85 g (4.48 mmol) of Boc-Ahx-Gly-Trp-Phe-OMe (XIII) and operating as described in Example 1, Step 4, but purifying the crude oily deblocked compound XIV with several washing with Et$_2$0, compound XIV (1.54 g, 60% yield) was eventually isolated from iPrOH/Et$_2$0. Rf$_D$ 0.75; E$_{1.2}$ 0.68.

Example 5

Preparation of HCl . H-Leu-Gly-Trp-Phe-OMe (XVI)

Step 1.   Boc-Leu-Gly-Trp-Phe-OMe   (XV)

Starting from 1.76 g (6.10 mmol) of Boc-Leu-Gly-OH (II) and 2.45 g (6.10 mmol) of HCl . H-Trp-Phe-OMe (XII), and operating as described in Example 3, Step 1, 3.10 g ( 80% yield) of compound XV  were obtained an oil: $Rf_C$ 0.73.

Step 2.   HCl . H-Leu-Gly-Trp-Phe-OMe   (XVI)

Starting from 2.75 g (4.30 mmol) of Boc-Leu-Gly-Trp-Phe-OMe (XV)   and operating as described in Example 1, Step 4, 2.09 g (85%  yield)  of compound XVI were obtained from i PrOH/$Et_2O$: m.p. 103-108°C (foam); $[\alpha]_D^{26}$ - 9.4° (c= 1 MeOH); $Rf_D$ 0.73; $E_{1.2}$ 0.67.

## C l a i m s

1. An animal growth promotant which comprises as an effective ingredient a peptide of the following formula I :

$$X-A-B-Trp-C-Y$$

wherein

X    is selected between hydrogen and t-butoxycarbonyl;

A    is selected from the group consisting of Leu, Ile, Phe, Ahx, Met, MetO and Phe(Cl), either of L- or D-configuration;

B    is selected from the group consisting of Pro, Ala, Gly, Val, Leu and Pip, either of L- or D-configuration;

C    is selected from the group consisting of Val, Leu, Met, MetO, Ile, Phe, Phe(Cl) and Ahx, either of L- or D-configuration; or A, B and C independently represent a chemical bond provided that at least one of them is an amino acid residue;

Y    is selected from the group consisting of OH, OMe, OEt, OBzl, $NH_2$, NH-Et, $NEt_2$
and the veterinarily acceptable salts thereof.

2. A feed composition for promoting the growth of growing animals, which comprises a sufficient amount of the peptide as defined in claim 1 and a compatible animal nutrition source.

3. A veterinary composition for subcutaneous implant comprising a peptide as defined in claim 1 in admixture with a veterinarily acceptable diluent or carrier.

4. A method for promoting the growth of growing animals, which comprises administering daily in their feed rations or by subcutaneous implant, an effective amount of a peptide as defined in claim 1, or a veterinarily acceptable salt thereof.

5. A method for improving the rate of weight of growing animals, which comprises administering daily in their feed rations, or by subcutaneous implant, an effective amount of a peptide as defined in claim 1 or a veterinarily acceptable salt thereof.

6. A method for improving the efficiency of feed utilization by growing animals, which comprises administering daily in their feed rations, or by subcutaneous implant, an effective amount of a peptide as defined in claim 1 or a veterinarily acceptable salt thereof.

7. The feed composition according to claim 2, wherein the concentration of the peptide in the feed rations is from about 0.1 to 100 mcg/Kg of feed.

8. The veterinary composition according to claim 3, wherein the release of the peptide per day is in a range from 0.1 to 300 ng/Kg.

9. A peptide of formula (I) as defined in claim 1, wherein either C is a chemical bond or A is different from L-Leu or L-Phe.

10. A peptide according to claim 9, which is selected from the group consisting of :

H-Ahx-Gly-Trp-Phe-OH

H-Ahx-Gly-Trp-Phe-OMe

H-Ahx-Gly-Trp-Phe-NH$_2$

H-Phe(Cl)-Gly-Trp-Phe-OH

H-Phe(Cl)-Gly-Trp-Phe-OMe

H-Phe(Cl)-Gly-Trp-Phe-NH$_2$

H-Ahx-Gly-Trp-Val-OH

H-Ahx-Gly-Trp-Val-OMe

H-Ahx-Gly-Trp-Val-NH$_2$

H-Ahx-Ala-Trp-Val-OH

H-Ahx-Ala-Trp-Val-OMe

H-Ahx-Ala-Trp-Val-NH$_2$

H-Ala-Trp-Met-OH

H-Ala-Trp-Met-OMe

H-Ala-Trp-Met-NH$_2$

H-Ala-Trp-Phe-OH

H-Ala-Trp-Phe-OMe

H-Ala-Trp-Phe-NH$_2$

H-Ala-Trp-Leu-OH

H-Ala-Trp-Leu-OMe

H-D-Leu-Gly-Trp-Met-OH

H-D-Leu-Gly-Trp-Met-OMe

H-D-Leu-Gly-Trp-Met-NH$_2$

H-D-Leu-Gly-Trp-Ahx-OH

H-D-Leu-Gly-Trp-Ahx-OMe

H-D-Leu-Gly-Trp-Ahx-NH$_2$

H-Phe(Cl)-Gly-Trp-Met-OH

H-Phe(Cl)-Gly-Trp-Met-OMe

H-Phe(Cl)-Gly-Trp-Met-NH$_2$

H-D-Phe-Gly-Trp-Met-OH

H-D-Phe-Gly-Trp-Met-OMe

H-D-Phe-Gly-Trp-Met-NH$_2$

H-Ahx-Gly-Trp-Met-OH

H-Ahx-Gly-Trp-Met-OMe

H-Ahx-Gly-Trp-Met-NH$_2$

H-Ala-Trp-Leu-NH$_2$

H-Ala-Trp-Val-OH

H-Ala-Trp-Val-OMe

H-Ala-Trp-Val-NH$_2$

H-D-Ala-Trp-Met-OH

H-D-Ala-Trp-Met-OMe

H-D-Ala-Trp-Met-NH$_2$

H-D-Ala-Trp-Met-NH$_2$

H-Leu-Gly-Trp-OH

H-Leu-Gly-Trp-OMe

H-Leu-Gly-Trp-NH$_2$

H-Leu-D-Ala-Trp-OH

H-Leu-D-Ala-Trp-OMe

H-Leu-D-Ala-Trp-NH$_2$

H-D-Leu-Gly-Trp-OH

H-D-Leu-Gly-Trp-OMe

H-D-Leu-Gly-Trp-NH$_2$

H-Ahx-Gly-Trp-OH

H-Ahx-Gly-Trp-OMe

H-Ahx-Gly-Trp-NH$_2$

H-Ahx-D-Ala-Trp-OH

H-Ahx-D-Ala-Trp-OMe

H-Ahx-D-Ala-Trp-NH$_2$

H-D-Ahx-Gly-Trp-OH

H-D-Ahx-Gly-Trp-OMe

H-D-Ahx-Gly-Trp-NH$_2$

11. A peptide according to claim 1, which is :

H-Leu-Gly-Trp-Met-OH

H-Leu-Pro-Trp-Met-OH

H-Leu-Pro-Trp-Met-OMe

H-Leu-Pro-Trp-Met-NH$_2$

H-Leu-Ala-Trp-Met-OH

H-Leu-Ala-Trp-Met-OMe

H-Leu-Ala-Trp-Met-NH$_2$

H-Leu-D-Ala-Trp-Met-OH

H-Leu-D-Ala-Trp-Met-OMe

H-Leu-D-Ala-Trp-Met-NH$_2$

H-Leu-Ala-Trp-Ahx-OH

H-Leu-Ala-Trp-Ahx-OMe

H-Leu-Ala-Trp-Ahx-NH$_2$

H-Leu-Gly-Trp-Phe-OH

·H-Leu-Gly-Trp-Phe-OMe

or  H-Leu-Gly-Trp-Phe-NH$_2$

12. A process for preparing a peptide of formula (I) as defined in claims 9 to 11, which comprises condensing a compound of formula II

X-A-B-OH

in the presence of a condensing agent such as dicyclohexylcarbodiimide, alone or together with a racemization preventing agent, or a mixed anhydride, activated ester or azide derivative of the compound of formula II as above defined, and a compound of formula III :

H-Trp-C-Y

wherein A,B,C,X and Y are as defined in claims 9 to 11'save that X does not represent a hydrogen atom and  Y does not represent a OH group,optionally converting a so obtained peptide of formula I into another peptide of formula I by esterification, hydrolysis or ammonolysis, and deprotecting if desired the resultant peptide.

## Claims for Austria

1. A process for preparing an animal growth promotant, characterized in that the following peptide according to formula I or the veterinarily acceptable salts thereof are synthesized:

$$X-A-B-Trp-C-Y \qquad (I)$$

wherein

X   is selected between hydrogen and t-butoxycarbonyl;

A   is selected from the group consisting of Leu, Ile, Phe, Ahx, Met, MetO and Phe(Cl), either of L- or D-configuration;

B   is selected from the group consisting of Pro, Ala, Gly, Val, Leu and Pip, either of L- or D-configuration;

C   is selected from the group consisting of Val, Leu, Met, MetO, Ile, Phe, Phe(Cl) and Ahx, either of L- or D-configuration; or A, B and C independently represent a chemical bond provided that at least one of them is an amino acid residue;

Y   is selected from the group consisting of OH, OMe, OEt, OBzl, $NH_2$, NH-Et, $NEt_2$.

2. A process for preparing a feed composition for promoting the growth of growing animals, characterized in that a sufficient amount of a peptide as defined in claim 1 is added to a compatible animal nutrition source.

3. A process for preparing a veterinary composition for subcutaneous implant, characterized in that a peptide as defined in claim 1 is admixed with a veterinarily acceptable diluent or carrier.

4. A method for promoting the growth of growing animals, which comprises administering daily in their feed rations or by subcutaneous implant, an effective amount of a peptide as defined in claim 1, or a veterinarily acceptable salt thereof.

5. A method for improving the rate of weight of growing animals, which comprises administering daily in their feed rations, or by subcutaneous implant, an effective amount of a peptide as defined in claim 1 or a veterinarily acceptable salt thereof.

6. A method for improving the efficiency of feed utilization by growing animals, which comprises administering daily in their feed rations, or by subcutaneous implant, an effective amount of a peptide as defined in claim 1 or a veterinarily acceptable salt thereof.

7. A process for preparing the feed composition according to claim 2, characterized in that the concentration of the peptide in the feed rations is selected from about 0.1 to 100 mcg/kg of feed.

8. A process for preparing a veterinary composition according to claim 3, wherein the release of the peptide per day is in a range from 0.1 to 300 ng/kg.

9. A process for preparing a peptide according to formula I as defined in claim 1, characterized in that a peptide is synthesized wherein either C is a chemical bond or A is different from L-Leu or L-Phe.

10. A process according to claim 9, characterized in that one of the following peptides is synthesized:

H-Ahx-Gly-Trp-Phe-OH

H-Ahx-Gly-Trp-Phe-OMe

H-Ahx-Gly-Trp-Phe-NH$_2$

H-Phe(Cl)-Gly-Trp-Phe-OH

H-Phe(Cl)-Gly-Trp-Phe-OMe

H-Phe(Cl)-Gly-Trp-Phe-NH$_2$

H-Ahx-Gly-Trp-Val-OH

H-Ahx-Gly-Trp-Val-OMe

H-Ahx-Gly-Trp-Val-NH$_2$

H-Ahx-Ala-Trp-Val-OH

H-Ahx-Ala-Trp-Val-OMe

H-Ahx-Ala-Trp-Val-NH$_2$

H-Ala-Trp-Met-OH

H-Ala-Trp-Met-OMe

H-Ala-Trp-Met-NH$_2$

H-Ala-Trp-Phe-OH

H-Ala-Trp-Phe-OMe

H-Ala-Trp-Phe-NH$_2$

H-Ala-Trp-Leu-OH

H-Ala-Trp-Leu-OMe

H-D-Leu-Gly-Trp-Met-OH

H-D-Leu-Gly-Trp-Met-OMe

H-D-Leu-Gly-Trp-Met-NH$_2$

H-D-Leu-Gly-Trp-Ahx-OH

H-D-Leu-Gly-Trp-Ahx-OMe

H-D-Leu-Gly-Trp-Ahx-NH$_2$

H-Phe(Cl)-Gly-Trp-Met-OH

H-Phe(Cl)-Gly-Trp-Met-OMe

H-Phe(Cl)-Gly-Trp-Met-NH$_2$

H-D-Phe-Gly-Trp-Met-OH

H-D-Phe-Gly-Trp-Met-OMe

H-D-Phe-Gly-Trp-Met-NH$_2$

H-Ahx-Gly-Trp-Met-OH

H-Ahx-Gly-Trp-Met-OMe

H-Ahx-Gly-Trp-Met-NH$_2$

H-Ala-Trp-Leu-NH$_2$

H-Ala-Trp-Val-OH

H-Ala-Trp-Val-OMe

H-Ala-Trp-Val-NH$_2$

H-D-Ala-Trp-Met-OH

H-D-Ala-Trp-Met-OMe

H-D-Ala-Trp-Met-NH$_2$

H-D-Ala-Trp-Met-NH$_2$

H-Leu-Gly-Trp-OH

H-Leu-Gly-Trp-OMe

H-Leu-Gly-Trp-NH$_2$

H-Leu-D-Ala-Trp-OH

H-Leu-D-Ala-Trp-OMe

H-Leu-D-Ala-Trp-NH$_2$

H-D-Leu-Gly-Trp-OH

H-D-Leu-Gly-Trp-OMe

H-D-Leu-Gly-Trp-NH$_2$

H-Ahx-Gly-Trp-OH

H-Ahx-Gly-Trp-OMe

H-Ahx-Gly-Trp-NH$_2$

H-Ahx-D-Ala-Trp-OH

H-Ahx-D-Ala-Trp-OMe

H-Ahx-D-Ala-Trp-NH$_2$

H-D-Ahx-Gly-Trp-OH

H-D-Ahx-Gly-Trp-OMe

H-D-Ahx-Gly-Trp-NH$_2$

11. A process according to claim 1, characterized in that one of the following compounds is synthesized:

H-Leu-Gly-Trp-Met-OH

H-Leu-Pro-Trp-Met-OH

H-Leu-Pro-Trp-Met-OMe

H-Leu-Pro-Trp-Met-NH$_2$

H-Leu-Ala-Trp-Met-OH

H-Leu-Ala-Trp-Met-OMe

H-Leu-Ala-Trp-Met-NH$_2$

H-Leu-D-Ala-Trp-Met-OH

H-Leu-D-Ala-Trp-Met-OMe

H-Leu-D-Ala-Trp-Met-NH$_2$

H-Leu-Ala-Trp-Ahx-OH

H-Leu-Ala-Trp-Ahx-OMe

H-Leu-Ala-Trp-Ahx-NH$_2$

H-Leu-Gly-Trp-Phe-OH

H-Leu-Gly-Trp-Phe-OMe  or

H-Leu-Gly-Trp-Phe-NH$_2$

12. A process for preparing a peptide of formula (I) as defined in claims 9 to 11, which comprises condensing a compound of formula II

$$X-A-B-OH \qquad (II)$$

in the presence of a condensing agent such as dicyclohexylcarbodiimide, alone or together with a racemization preventing agent, or a mixed anhydride, activated ester or azide derivative of the compound of formula II as above defined, and a compound of formula III :

$$H-Trp-C-Y$$

wherein A,B,C,X and Y are as defined in claims 9 to 11'save that X does not represent a hydrogen atom and Y does not represent a OH group,optionally converting a so obtained peptide of formula I into another peptide of formula I by esterification, hydrolysis or ammonolysis, and deprotecting if desired the resultant peptide.